# EUROPEAN PATENT SPECIFICATION

(11) **EP 0 663 810 B1**
(45) Date of publication and mention of the grant of the patent: **18.11.1998**
(21) Application number: 93915745.9
(22) Date of filing: 29.06.1993
(51) Int. Cl.: A61F 2/32, A61F 2/34

(54) **LUBRICATED CUP FOR TOTAL JOINT PROSTHESIS**
GESCHMIERTE PFANNE FÜR GESAMTGELENKPROTHESE
CUPULE LUBRIFIEE POUR PROTHESE COMPLETE DE L'ARTICULATION COXO-FEMORALE

(43) Date of publication of application: 26.07.1995
(73) Proprietor: AO-FORSCHUNGSINSTITUT DAVOS, 7270 Davos-Platz (CH)
(72) Inventor: TEPIC, Slobodan, CH-7270 Davos (CH)
(74) Representative: Lusuardi, Werther Giovanni, Dr.
(86) International application number: EP9301678
(87) International publication number: WO9501139

(56) References cited:
- EP-A- 0 084 094
- EP-A- 0 253 941
- EP-A- 0 444 382
- DE-A- 2 139 878
- DE-A- 3 343 863
- US-A- 3 864 758

## Description

### FIELD OF THE INVENTION

This invention relates to a total joint prosthesis, in particular to the total hip prosthesis, provided by dynamic lubrication means in order to reduce the wear of the articular components. The mechanism mimics that of the "weeping" lubrication in natural cartilage.

### BACKGROUND ART

Low friction arthoplasty was developed by Sir J. Charnley in early sixties - over the last three decades millions of patients have been relieved of suffering from joint degenerating illnesses. Total joint replacement has truly revolutionized the field of orthopaedics. After an unsuccessful attempt to use the metal head on Teflon cup combination, Charnley developed the metal head on UHMWPE (Ultra High Molecular Weight Polyethylene) cup combination, which remains a standard option today. Lubrication is of the boundary type and depends mostly on the properties of UHMWPE. The most common materials for the prosthesis head are chrome-based alloys. Ceramic heads are an alternative, and seem to reduce wear of the polymer cup by factor two. UHMWPE wear against a metal head of about 0.2 mm/year is not a duration limiting factor in itself (usual cup thickness is about 8mm, sufficient for 40 years of wear). However, numerous studies support the opinion that wear particles (some 400 billion / year) are a contributing factor to prosthesis loosening by indirect mechanisms of bone resorption - these particles can be traced to all surrounding tissues including bone. A broad consensus in the field of total joint replacement is that polymer cup wear is a serious problem. Modern manufacturing technologies, and specifically new surface treatments and finishes have revised the almost forgotten metal-on-metal approach. Ceramic-on-ceramic has also been tried, but none of the alternatives have shown the predictably good results of the plastic cup. None of the alternatives has offered a mode of lubrication which could be rationally developed to meet the demanding task of, for example, human hip joint.

From DE-A-2 139 878 and also from DE-A-2 33 43 863 a hip joint prosthesis is known having a fluid filled compartment in the ball head connected to the interarticular gap formed with the cooperating rigid acetabular socket.
In DE-A-2 33 43 863 the fluid filled compartment is additionally provided with a piston-in-cylinder pumping device.
The disadvantages of this known device are the incomplete sealing of the fluid filled compartment and the production of wear particles.

This invention is aimed at solving the problem of lubricating a total joint. The design is motivated by the mechanism of lubrication of natural cartilage - known as "weeping" after C. McCutchen. The goal is to allow the joint force to pressurize a fluid filled container and pump the fluid into the interarticular gap. At best, all of the load would by supported by the fluid film ("weeping" from the container). The pressures needed to support the loads in , for example, a hip prosthesis are in excess of 5MPa (50 bars, or atm). The fluid is to be refilled during the period when the load is small and the container is expanded to suck back the fluid lost in the high load phase. If only suction is used to bring the fluid back, the maximum pressure difference driving the fluid is 0.1MPa. In locomotion the high load phase is of about the same duration as the low load phase. To maintain the flow during most of the high load phase, the same amount of fluid should be sucked in during the low load phase (lubricating fluid is the fluid found around the joint after joint replacement which resembles synovial fluid). With the big difference in driving pressures (some fifty times) this can only be done by changing the resistance to flow between the two phases. The preferred way of modulating the resistance to flow through the interarticular gap is by deforming the cup under load, so as to reduce the gap under higher loads. The cup is made thin enough to deform at the equator by the desired amount - the deformation is imposed by suspension of the cup within an outer shell facing the bone. The fluid compartment is preferably interposed between the articular cup and the outer shell and is pressurized through a membrane spanned between the cup and the outer shell. Alternatively, the fluid compartment is placed inside the head/neck of the prosthesis. The advantage of this approach is that the modulation of the resistance by cup deformation can be fully elastic, i.e. instantaneous. In that way no fluid is lost during the closing of the gap. The fluid volume can also be increased by using a simple piston type pump between the head and the neck of the prosthesis.

### BRIEF DESCRIPTION OF THE DRAWINGS

In the drawings:
Figure 1 is a cross section of the lubricated cup with the integral fluid compartment;
Figure 2 shows exudation of the fluid during the high load phase of the joint cycle;
Figure 3 shows refilling of the fluid compartment during the low load phase of the joint cycle;
Figure 4 shows a cup with grooves to concentrate the pressure drop at the equatorial section of the cup;
Figure 5 is a cross section of the prosthesis head with the integral fluid compartment;
Figure 6 shows the elastic cup articulated against the prosthesis head with the fluid compartment;
Figure 7 is a cross section of the lubricated cup and the head with the piston-in-cylinder type of the fluid compartment.

### DETAILED DESCRIPTION OF THE INVENTION

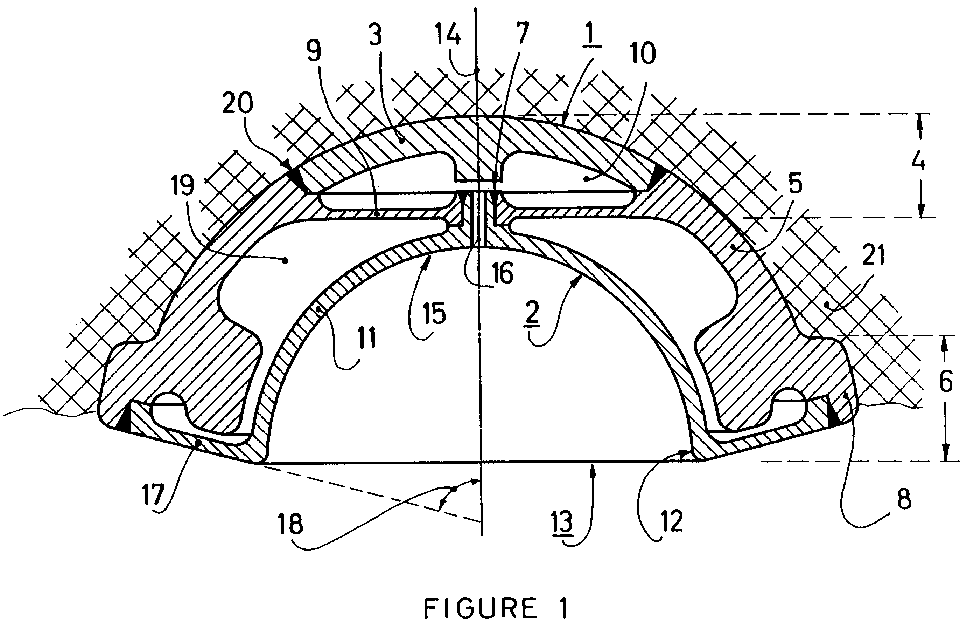

Figure 1 shows a cross-section of the socket of a total hip joint prosthesis according to the invention. The socket is fixed to the bone 21 by either bone cement or bone-ingrowth interface. Bone facing surface of the socket is denoted by 1. The spherical cap 11 glides on the head of the prosthesis on its articular surface 2. The main body of the socket consists of the first exterior wall member 3, and the second exterior wall member 5. The main body at its peripheral region 6 supports the cup 11 by a conically shaped shell 17. The shell 17 meets the cup 11 along the equator 12 and is welded to the main part of the socket 5 at its peripheral rim 8. The angle 18 of the conical section 17 is smaller than 90 degrees. In the apex region of the socket 4, the exterior wall member 5 meets the membrane 9 of the fluid compartment 10. The membrane 9 has a central opening 7 where it is welded to the cup 11. At the polar region 15 the cup has a central bore 16 through which the fluid compartment 10 communicates with the interarticular gap (the gap between the cup and the head of the prosthesis). Equatorial plane of the socket is depicted by 13, while the polar axis of symmetry is depicted by 14. The space 19 enclosed by the cup 11, the body member 5, the membrane 9, and the shell 17 is filled by gas only. The fluid compartment 10 is exteriorly sealed by the weld 20 between the exterior wall members 3 and 5.

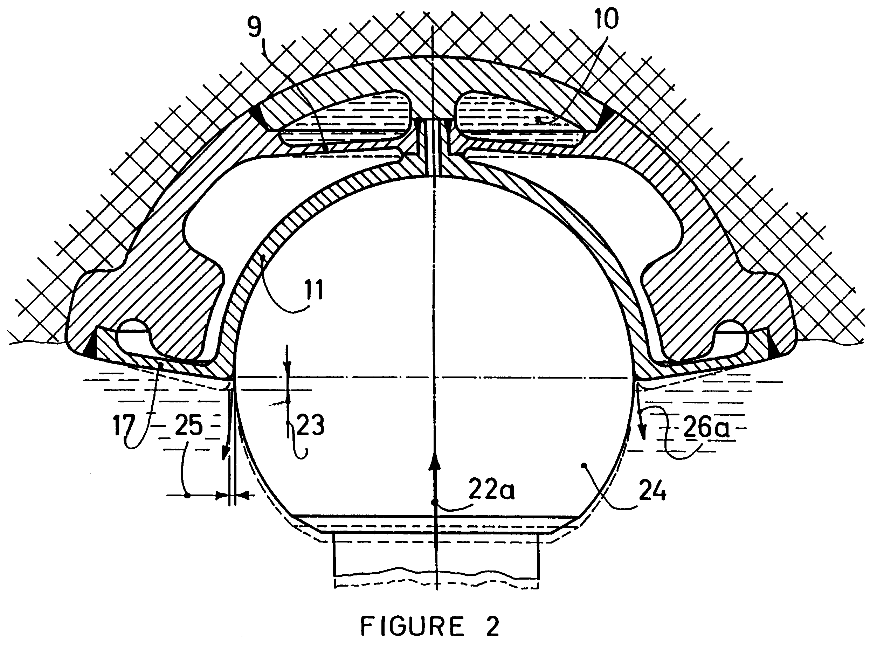

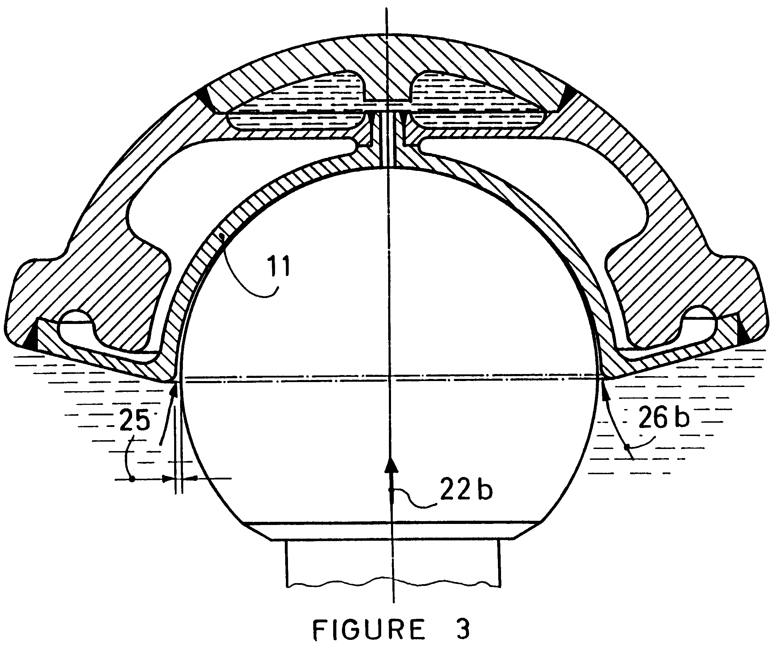

Figure 2 shows the high load phase of the joint cycle. The joint force 22a pushes the head of the prosthesis 24 into the socket and causes inward axial movement 23 of the cup 11. The inward axial movement 23, due to the coupling of the conical shell 17 to the cup 11, causes reduction of the initial gap 25 between the head 24 and the cap 11. Thus the fluid from the compartment 10, pressurized by the membrane 9, is forced out through a tight interarticular space as shown by the arrow 26a. The goal is not to loose more fluid than can be sucked back in during the low load part of the cycle which is shown on Figure 3. Now the joint force 22b is low and allows the restoring force of the shells 11 and 17 and the membrane 9 to push the head out of the cup 11 and open the gap 25. With no more than 0,1MPa (1 bar) pressure difference (if the fluid compartment were at vacuum) the fluid is sucked back in as shown by arrow 26b. The resistance to flow through the open gap must be reduced by about the same ratio as the driving pressures, i.e. factor 50 to 100.

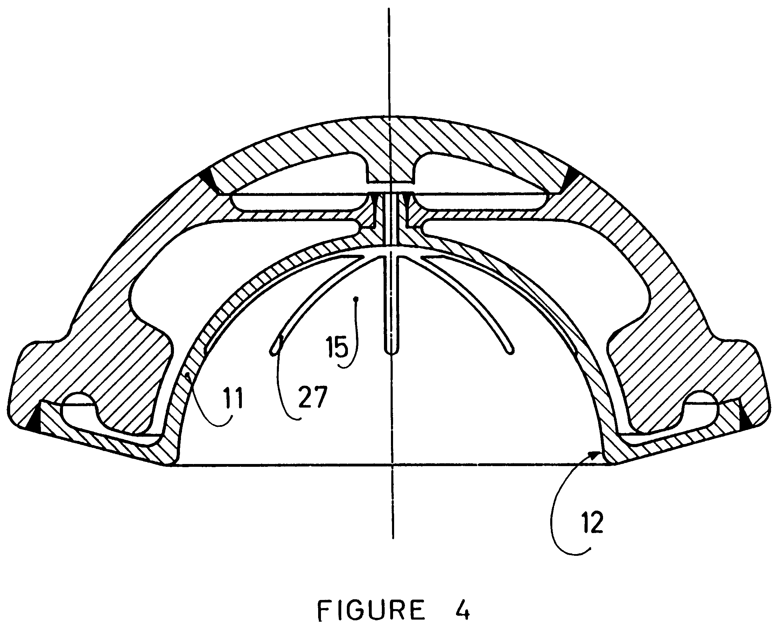

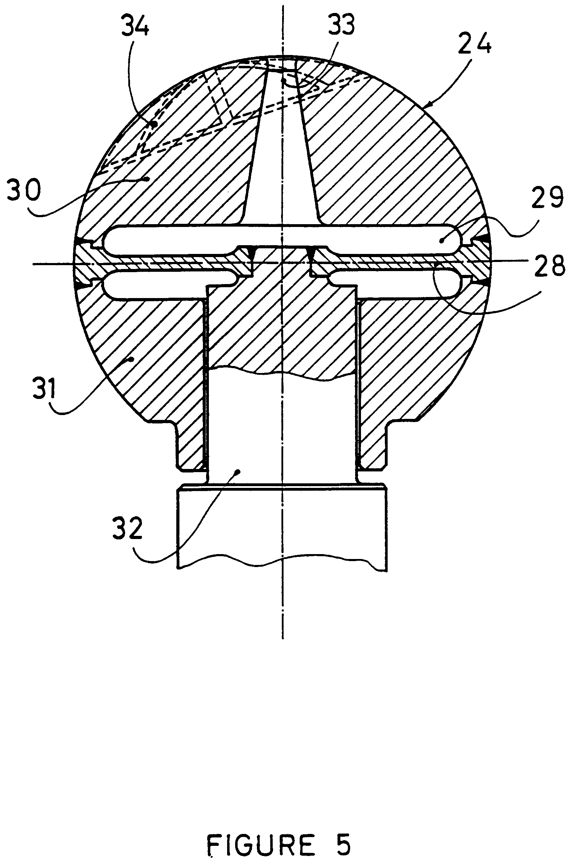

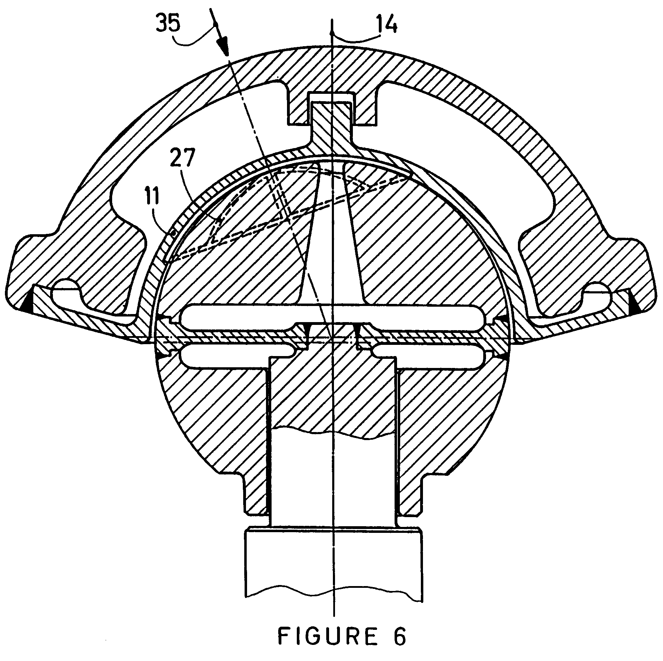

Figure 4 shows some grooves 27 cut into the cup 11 in the polar region . The purpose of the grooves is to concentrate the pressure drop of the fluid flow into equatorial region 12 of the cup where resistance modulation due to cup deformation is strongest. In that way getting the factor of 50 to 100 in the flow resistance out to flow resistance in should be easier to accomplish. AS shown on Figure 6 these grooves can be used to choose the area of high pressure to better balance the joint force. The grooves can alternatively be cut into the head 24 as shown on Figure 5.

Figure 5 shows an alternative placement of the pump inside the head 24. The upper part of the head 30 is welded onto membrane 28, which is welded onto lower part of the head 31. The membrane is also welded onto the neck 32 of the prosthesis. The hole 33 connects the fluid compartment 29 to the interarticular gap.

Figure 6 shows the pump head against a deformable cup 11. The advantage of placing the pump into the head is that resistance modulation is fast now - with the pump inside the socket some fluid had to be pushed out before the gap would close. The joint force 35 may not be aligned with the polar head axis 14. Using the grooves 27 centered onto force axis should give better balance of the fluid support. Alternatively, the grooves could be cut into the head as shown by 34 on Figure 5.

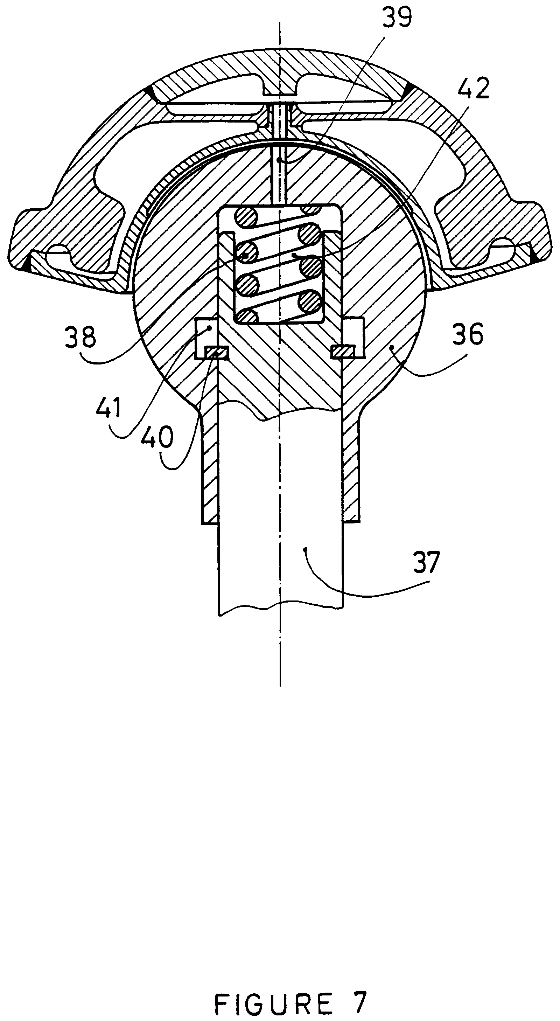

Figure 7 is a cross section of the prosthetic joint with yet another type of pump placed between the head of the prosthesis 36 and the neck 37. The movement of the head 36 on the neck 37 under joint loading pressurizes fluid in the compartment 42 and forces the fluid through the hole 39 into interarticular gap. When the force is reduced the spring 38 pushes the neck out again and refills the compartment 42. Movement of the snap ring 40 within the groove 41 limits the movement of the head 36 on the neck 37. Some leakage of the fluid will occur between the neck and the head, but this can be compensated by the generous stroke possible with this construction.

Wear at the head/ neck interface is much easier to control than at the spherical joint - congruency of a cylindrical bearing is superior to that of a spherical bearing. Additionally, only a smaller component of the joint force acts normal to the axis of the neck.

## Claims

1. A dynamically lubricated total joint prosthesis comprising
A) two articulating members,
aa) a cup member (11) having an equatorial and a polar region and
ab) a head member (24;31;36) attached to the neck (32;37) of the prosthesis and cooperating with the cup member (11); and
wherein
B) at least one of the two articulating members is movably supported on the corresponding bone-anchored parts (3,5;50) of the prosthesis;
C) at least one fluid filled compartment (10;29;41) is connected to the interarticular gap (25) between the two articulating members; and
D) the joint load reduces the volume of said fluid filled compartment (10;29;41) and increases the pressure therein;
**characterized in that**
E) the cup member (11) of the prosthesis is deformable so that higher joint load leads to reduction of said interarticular gap (25) in the equatorial region of the cup member (11).

2. A total joint prosthesis according to claim 1, characterized in that the cup member (11) is supported along its equatorial region by a conically shaped shell (17) inside the bone-anchored socket (5) of the cup member (11).

3. A total joint prosthesis according to claim 1 or 2, characterized in that the cup member (11) has grooves (27) in said polar region.

4. A total joint prosthesis according to claim 1 or 2, characterized in that the head member (24;31;36) is provided with grooves (34).

5. A total joint prosthesis according to one of the claims 1 to 4, characterized in that the fluid filled compartment (10) is interposed between the cup member (11) and the bone-anchored socket (5) of said prosthesis.

6. A total joint prosthesis according to one of the claims 1 to 4, characterized in that the fluid filled compartment (29) is interposed between the head member (24;31;36) and the neck (32;37) of said prosthesis.

7. A total joint prosthesis according to claim 6, characterized in that the fluid filled compartment (29) is pressurized by a deformable membrane (28).

## Patentansprüche

1. Eine dynamisch geschmierte Gesamtgelenkprothese, welche
A) zwei Gelenkelemente,
aa) ein Schalenelement (11) mit einer äquatorialen und einer polaren Region und
ab) ein Kopfelement (24;31;36), welches an dem Hals (32;37) einer Prothese anbringbar ist, und mit dem Schalenelement (11) Zusammenwirkt; umfasst und
wobei
B) zumindest eines der zwei zusammenwirkenden Elemente auf den entsprechenden im Knochen verankerten Teilen (3;5;50) der Prothese bewegbar unterstützt ist;
C) zumindest ein fluidgefülltes Abteil (10;29;41) mit dem Gelenkspalt (25) zwischen den zwei Gelenkelementen verbunden ist; und
D) die Gelenkbelastung das Volumen des fluidgefüllten Abteils (10;29;41) reduziert und den Druck darin erhöht;
dadurch gekennzeichnet, dass
E) das Schalenelement (11) der Prothese deformierbar ist, so dass eine höhere Gelenkbelastung zu einer Reduktion des Gelenkspaltes (25) in der äquatorialen Region des Schalenelementes (11) führt.

2. Gesamtgelenkprothese nach Anspruch 1, dadurch gekennzeichnet, dass das Schalenelement (11) entlang seiner äquatorialen Region durch eine konische Hülse (17) innerhalb des im Knochen verankerten Sockels (5) des Schalenelementes (11) unterstützt ist.

3. Gesamtgelenkprothese nach Anspruch 1 oder 2, dadurch gekennzeichnet, dass das Schalenelement (11) in der polaren Region Rillen (27) aufweist.

4. Gesamtgelenkprothese nach Anspruch 1 oder 2, dadurch gekennzeichnet, dass das Kopfelement (24;31;36) Rillen (34) aufweist.

5. Gesamtgelenkprothese nach einem der Ansprüche 1 bis 4, dadurch gekennzeichnet, dass das fluidgefüllte Abteil (10) zwischen dem Schalenelement (11) und dem im Knochen verankerten Sockel (5) der Prothese eingeschoben ist.

6. Gesamtgelenkprothese nach einem der Ansprüche 1 bis 4, dadurch gekennzeichnet, dass das fluidgefüllte Abteil (10) zwischen dem Schalenelement (11) und dem Hals (32;37) der Prothese eingeschoben ist.

7. Gesamtgelenkprothese nach Anspruch 6, dadurch gekennzeichnet, dass das fluidgefüllte Abteil (10) durch eine deformierbare Membran (28) mit Druck beaufschlagt wird.

## Revendications

1. Prothèse d'articulation complète à lubrification dynamique, comportant:
A) deux éléments d'articulation,
aa) un élément en coupelle (11) présentant une région équatoriale et une région polaire, et
ab) un élément de tête (24; 31; 36) fixé au col (32; 37) de la prothèse et coopérant avec l'élément en coupelle (11); et dans laquelle
B) au moins l'un des deux éléments d'articulation est soutenu à déplacement sur les parties correspondantes (3, 5; 50) ancrées dans l'os de la prothèse;
C) au moins un compartiment (10; 29; 41) rempli de fluide est relié à l'interstice interarticulaire (25) formé entre les deux éléments d'articulation; et
D) la charge exercée sur l'articulation réduit le volume dudit compartiment (10; 29; 41) rempli de fluide et augmente la pression qui y règne,
caractérisée en ce que
E) l'élément en coupelle (11) de la prothèse est déformable de telle sorte qu'une charge plus élevée sur l'articulation entraîne une réduction dudit interstice interarticulaire (25) dans la région équatoriale de l'élément en coupelle (11).

2. Prothèse d'articulation complète selon la revendication 1, caractérisée en ce que l'élément en coupelle (11) est soutenu dans sa région équatoriale par une coquille (17) de forme conique située à l'intérieur de la douille (5), ancrée dans l'os, de l'élément en coupelle (11).

3. Prothèse d'articulation complète selon la revendication 1 ou 2, caractérisée en ce que l'élément en coupelle (11) présente des rainures (27) dans ladite région polaire.

4. Prothèse d'articulation complète selon les revendications 1 ou 2, caractérisée en ce que l'élément de tête (24; 31; 36) est doté de rainures (34).

5. Prothèse d'articulation complète selon l'une des revendications 1 à 4, caractérisée en ce que le compartiment (10) rempli de fluide est disposé entre l'élément en coupelle (11) et la douille (5) ancrée dans l'os de ladite prothèse.

6. Prothèse d'articulation complète selon l'une des revendications 1 à 4, caractérisée en ce que le compartiment (29) rempli de fluide est disposé entre l'élément de tête (24; 31; 36) et le col (32; 37) de ladite prothèse.

7. Prothèse d'articulation complète selon la revendication 6, caractérisée en ce que le compartiment (29) rempli de fluide est mis sous pression par une membrane déformable (28).
